# DEMANDE DE BREVET EUROPEEN

(11) **EP 1 557 412 A1**
(43) Date de publication de la demande: **27.07.2005**
(21) Numéro de dépôt: 05290160.0
(22) Date de dépôt: 25.01.2005
(51) Int. Cl.: C07D 285/24, A61K 31/549, A61P 25/28

(54) **Dérivés de benzothiadazines fluorées et leur utilisation comme modulateurs des récepteurs AMPA**

(30) Priorité: 26.01.2004 FR 0400689
(71) Demandeur: Les Laboratoires Servier, 92415 Courbevoie Cedex (FR)
(72) Inventeur: Francotte Pierre, 4000 Liege (BE); Fraikin Pierre, 4820 Dison (BE); Tullio Pascal, 4020 Jupille (BE); Pirotte Bernard, 4680 oUPEYE (BE); Lestage Pierre, 78170 La celle Saint Cloud (FR); Danober Laurence, 78360 Montesson (FR); Caignard Daniel-Henri, 78360 Le PECQ (FR); Renard Pierre, 78150 Le Chesnay (FR)

(57) **Abrégé**

Composé de formule (I) : 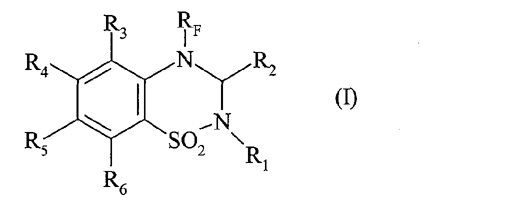 dans laquelle :
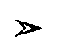 R_{F} représente un groupement mono- ou poly- fluoroalkyle, ou monofluoro- ou polyfluoro- cycloalkylalkyle,
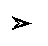 R₁ représente un atome d'hydrogène ou un groupement choisi parmi alkylaminocarbonyle et alkyle éventuellement substitué,
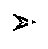 R₂ représente un atome d'hydrogène ou d'halogène ou un groupement choisi parmi cycloalkyle et alkyle éventuellement substitué,
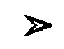 R₃ à R₆, identiques ou différents, représentent chacun un atome ou un groupement choisi parmi les atomes d'hydrogène, d'halogène et les groupements nitro, cyano, alkylsulfonyle, hydroxy, alkoxy, alkyle éventuellement substitué, et amino éventuellement substitué,
ainsi que leurs sels d'addition avec un acide ou une base pharmaceutiquement acceptable, et leurs isomères optiques lorsqu'ils existent.

## Description

La présente invention concerne de nouveaux dérivés de benzothiadiazines fluorées, leur procédé de préparation, les compositions pharmaceutiques qui les contiennent ainsi que leur utilisation en tant que modulateurs allostériques positifs des récepteurs AMPA.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, certains travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Des dérivés de benzothiadiazines ont été décrits en tant que modulateurs des récepteurs AMPA dans les demandes de brevet WO 98/12185 et WO 01/40210.

Il était particulièrement intéressant de synthétiser de nouveaux composés modulateurs des récepteurs AMPA afin d'augmenter la puissance, la sélectivité et la biodisponibilité des composés déjà décrits.

Plus spécifiquement, la présente invention concerne les composés de formule (I) : 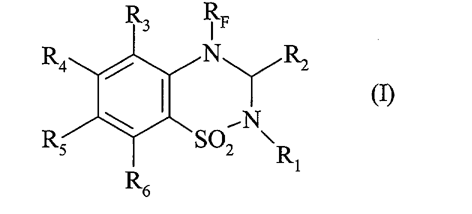 dans laquelle :
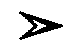 R_{F} représente un groupement mono- ou poly- fluoroalkyle C₁-C₆ linéaire ou ramifié, ou un groupement monofluoro- ou polyfluoro- cycloalkylalkyle, dans lequel la partie alkyle est en C₁-C₆ et est linéaire ou ramifiée, et la partie cycloalkyle est en C₃-C₇,
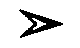 R₁ représente un atome d'hydrogène ou un groupement choisi parmi alkylaminocarbonyle C₁-C₆ linéaire ou ramifié et alkyle C₁-C₆ linéaire ou ramifié éventuellement substitué par un ou plusieurs atomes d'halogène, préférentiellement de fluor,
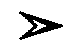 R₂ représente un atome d'hydrogène ou d'halogène ou un groupement choisi parmi cycloalkyle C₃-C₇ et alkyle C₁-C₆ linéaire ou ramifié éventuellement substitué par un ou plusieurs atomes d'halogène,
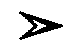 R₃, R₄, R₅ et R₆, identiques ou différents, représentent chacun un atome ou un groupement choisi parmi les atomes d'hydrogène, d'halogène et les groupements nitro, cyano, alkylsulfonyle C₁-C₆ linéaire ou ramifié, hydroxy, alkoxy C₁-C₆ linéaire ou ramifié, alkyle C₁-C₆ linéaire ou ramifié éventuellement substitué par un ou plusieurs atomes d'halogène, et amino éventuellement substitué par un ou deux groupements alkyle C₁-C₆ linéaire ou ramifié,
ainsi que leurs sels d'addition avec un acide ou une base pharmaceutiquement acceptable, et leurs isomères optiques lorsqu'ils existent,
étant entendu que R₃ représente un atome d'hydrogène lorsque R₆ ne représente pas un atome d'hydrogène.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique, camphorique.

Parmi les bases pharmaceutiquement acceptables, on peut citer à titre non limitatif l'hydroxyde de sodium, l'hydroxyde de potassium, la triéthylamine, la tertbutylamine.

Les groupements R_{F} préférés sont les groupements monofluoroalkyle C₁-C₆ linéaire ou ramifié, et plus particulièrement les groupements fluorométhyle, 2-fluoroéthyle, 3-fluoropropyle et 4-fluorobutyle.
Les groupements polyfluoroalkyle préférés sont les groupements 2,2-difluoroéthyle et 2,2,2-trifluoroéthyle.

R₁, R₂ et R₃ représentent chacun préférentiellement un atome d'hydrogène.
Lorsque R₂ représente un atome d'halogène, il représente préférentiellement un atome de fluor.

R₄ et R₆ représentent chacun préférentiellement un atome d'hydrogène ou d'halogène.
R₅ représente préférentiellement un atome d'halogène.

Sont plus particulièrement préférés les composés pour lesquels R₄ représente un atome d'hydrogène et R₅ représente un atome d'halogène, et les composés pour lesquels R₄ et R₅ représentent chacun un atome d'halogène.

Les composés préférés selon l'invention sont :
- le 7-chloro-4-(2-fluoroéthyl)-3,4-dihydro-2*H*-1-1,2,4-benzothiadiazine 1,1-dioxyde ;
- le 6,7-dichloro-4-(2-fluoroéthyl)-3,4-dihydro-2*H*-1,2,4-benzothiadiazine 1,1-dioxyde,
- le 6-chloro-7-fluoro-4-(2-fluoroéthyl)-3,4-dihydro-2*H*-1,2,4-benzothiadiazine 1,1-dioxyde, et
- le 6-chloro-7-bromo-4-(2-fluoroéthyl)-3,4-dihydro-2*H*-1,2,4-benzothiadiazine 1,1-dioxyde.

L'invention s'étend également au procédé de préparation des composés de formule (I) à partir du composé de formule (II) : 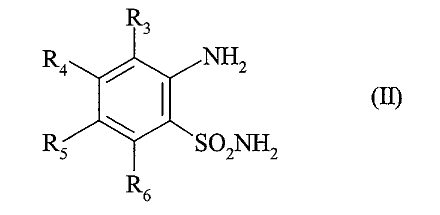 dans laquelle R₃, R₄, R₅ et R₆ sont tels que définis dans la formule (I),
que l'on cyclise en présence d'un composé de formule (III) :

R₂-C(OR₇)₃ (III)

dans laquelle R₂ est tel que défini dans la formule (I), et R₇ représente un groupement alkyle C₁-C₆ linéaire ou ramifié,
pour conduire au composé de formule (IV) : 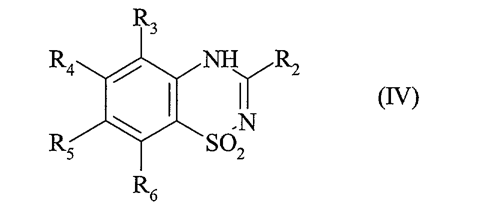 dans laquelle R₂, R₃, R₄, R₅ et R₆ sont tels que définis précédemment,
que l'on met en réaction avec un composé de formule (V) :

X ― R_{F} (V)

dans laquelle R_{F} est tel que défini dans la formule (I) et X représente un groupement partant choisi parmi les atomes d'iode, de brome et les groupements tosylate, mésylate et triflate,
pour conduire au composé de formule (VI) : 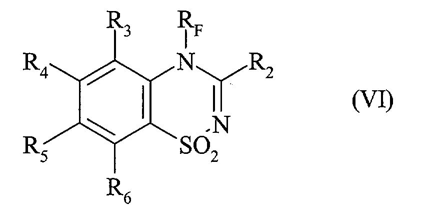 dans laquelle R₂, R₃, R₄, R₅, R₆ et R_{F} sont tels que définis précédemment,
que l'on met en réaction avec un agent réducteur, pour conduire au composé de formule (Ia), cas particulier des composés de formule (I) pour lequel R₁ représente un atome d'hydrogène : 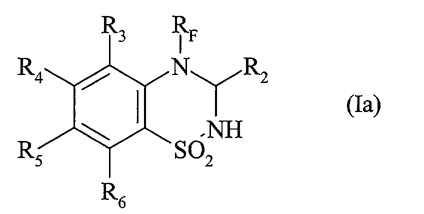 dans laquelle R₂, R₃, R₄, R₅, R₆ et R_{F} sont tels que définis précédemment,
composé de formule (Ia) que l'on met en réaction, lorsqu'on souhaite accéder aux composés de formule (I) pour lesquels R₁ représente un groupement alkyle éventuellement substitué,
avec un composé de formule (VII) :

X - R'₁ (VII)

dans laquelle R'₁ représente un groupement alkyle C₁-C₆ linéaire ou ramifié éventuellement substitué par un ou plusieurs atomes d'halogène, et X représente un groupement partant choisi parmi les atomes d'iode, de brome et les groupements tosylate, mésylate et triflate,
pour conduire au composé de formule (Ib) : 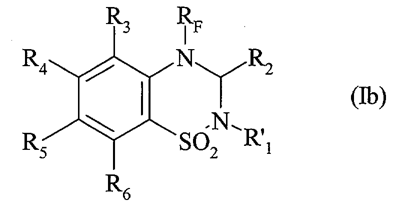 dans laquelle R₂, R₃, R₄, R₅, R₆, R_{F} et R'₁ sont tels que définis précédemment,
ou composé de formule (Ia) que l'on met en réaction, lorsqu'on souhaite accéder aux composés de formule (I) pour lesquels R₁ représente un groupement alkylaminocarbonyle,
avec un composé de formule (VIII) :

R₈-N=C=O (VIII)

dans laquelle R₈ représente un groupement alkyle C₁-C₆ linéaire ou ramifié,
pour conduire au composé de formule (Ic) : 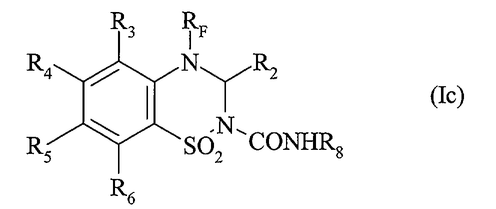 dans laquelle R₂, R₃, R₄, R₅, R₆, R_{F} et R₈ sont tels que définis précédemment,
les composés de formule (Ia), (Ib) et (Ic) constituant la totalité des composés de formule (I).

Le composé de formule (II) peut être obtenu, soit selon le procédé décrit par Girard et coll. (J. Chem. Soc. Perkin I 1979, 1043-1047), soit par chlorosulfonation d'une aniline appropriée à l'aide d'acide chlorosulfonique, suivie d'une conversion du chlorure de sulfonyle obtenu en sulfonamide correspondant à l'aide d'ammoniaque.

Les composés de formule (VI) sont nouveaux et font également partie de l'invention à titre d'intermédiaires de synthèse des composés de formule (I).

Les composés de formule (VIa), cas particulier des composés de formule (VI) pour lesquels R_{F} représente un groupement mono- ou polyfluoroalkyle C₂-C₆ : 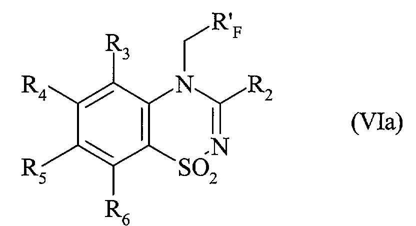 dans laquelle R₂, R₃, R₄, R₅, R₆ sont tels que définis dans la formule (I), et R'_{F} représente un groupement mono- ou polyfluoroalkyle C₁-C₅,
peuvent également être préparés par acylation du composé de formule (II) en présence d'un composé de formule (IX) :

R'_{F}-COCl (IX)

dans laquelle R'_{F} est tel que défini précédemment,
pour conduire au composé de formule (X) : 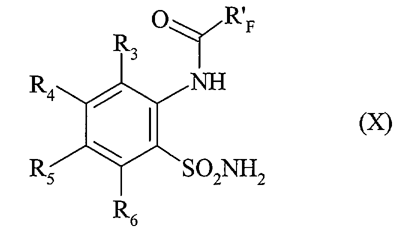 dans laquelle R₃, R₄, R₅, R₆ et R'_{F} sont tels que définis précédemment,
que l'on met en réaction avec de l'hydrure de lithium aluminium, pour conduire au composé de formule (XI) : 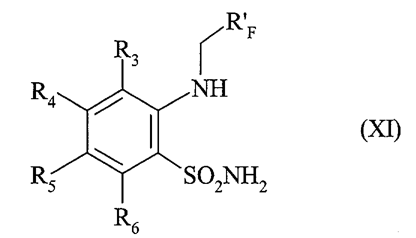 dans laquelle R₃, R₄, R₅, R₄ et R'_{F} sont tels que définis précédemment,
que l'on cyclise en présence d'un composé de formule (III), pour conduire au composé de formule (VIa).

Les composés de la présente invention présentent des propriétés activatrices des récepteurs AMPA qui les rendent utiles dans le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéfiés, les comprimés sublinguaux, les gélules, les tablettes, les suppositoires, les crèmes, les pommades, les gels dermiques, les préparations injectables, et les suspensions buvables.

La posologie utile est adaptable selon la nature et la sévérité de l'affection, la voie d'administration ainsi que l'âge et le poids du patient. Cette posologie varie de 0,01 à 500 mg par jour en une ou plusieurs prises.

Les exemples suivants illustrent l'invention.

Les produits de départ utilisés sont des produits connus ou préparés selon des modes préparatoires connus.

Les structures des composés décrits dans les exemples ont été déterminées selon les techniques spectrophotométriques usuelles (infrarouge, RMN, spectrométrie de masse).

### EXEMPLE 1 : :7-Chloro-4-(2-fluoroéthyl)-3,4-dihydro-2H-1,2,4-benzothiadiazine 1,1-dioxyde

### Stade A : 7-Chloro-4H-1,2,4-benzothiadiazine 1,1-dioxyde

A 4,75 g de 2-amino-5-chlorobenzènesulfonamide, dont la préparation est décrite dans le journal J. Chem. Soc. Perkin 1 1979, 1043-1047, sont ajoutés 40 ml d'orthoformiate d'éthyle. Le mélange est porté à 110°C pendant 30 minutes en vase ouvert. On observe une mise en solution, puis l'apparition d'un précipité.
Le volume du milieu réactionnel est ensuite réduit de moitié sous dépression. Le précipité obtenu est recueilli par filtration, puis lavé à l'éther et séché, pour conduire au produit attendu sous forme de cristaux blancs avec un rendement de 85 à 90 %.
*Point de fusion* *: 243-244°C*

### Stade B : 7-Chloro-4-(2 fluoroéthyl)-4H-1,2,4-benzothiadiazine 1, 1 -dioxyde

600 mg du composé obtenu au stade précédent sont dissous à chaud dans 15 ml d'acétonitrile, puis 1,2 g de carbonate de potassium et 0,6 ml de 1-fluoro-2-iodoéthane sont ajoutés et le milieu réactionnel est chauffé à 70°C en enceinte hermétique sous agitation pendant 30 heures. Les solvants sont alors éliminés sous pression réduite. Le résidu solide ainsi obtenu est repris par 5 ml d'eau et est immédiatement recueilli sur filtre. Le contenu du filtre est lavé plusieurs fois à l'eau, séché, puis mis en suspension dans un minimum d'acétate d'éthyle et recueilli sur filtre. La poudre ainsi obtenue est recristallisée dans l'acétone chaude, pour conduire en produit du titre avec un rendement de 75-80 %.
*Point de fusion* *: 211-218°C*

### Stade C: 7-Chloro-4-(2-fluoroéthyl)-3,4-dihydro-2H-1,2,4-benzothiadiazine 1,1-dioxyde

A 500 mg du composé obtenu au stade précédent en solution dans 25 ml d'isopropanol sont ajoutés 750 mg de borohydrure de sodium, puis le mélange est chauffé à 50°C pendant 40 min. Le solvant est ensuite éliminé sous pression réduite, puis le résidu obtenu est repris par 10 ml d'eau, refroidi dans un bain de glace et amené à pH = 5 par ajout d'acide chlorhydrique concentré. Après extraction au chloroforme, les phases organiques réunies sont séchées, filtrées et évaporées, et le résidu obtenu est cristallisé dans le méthanol, pour conduire après filtration puis séchage au produit attendu, avec un rendement de 85 %.
*Point de fusion**: 120-121* °C

### EXEMPLE 2 : 7-Chloro-4-(2-fluoroéthyl)-2-méthyl-3-4-dihydro-2H-1,2,4-benzothiadiazine 1,1-dioxyde

360 mg du composé de l'exemple 1 sont placés dans 8 ml d'acétonitrile. 600 mg de carbonate de potassium sont ajoutés ainsi que 0,5 ml d'iodure de méthyle. La solution est chauffée à 55°C pendant 5h. Le solvant est ensuite éliminé sous pression réduite et le résidu est placé en suspension dans 5 ml d'eau. L'insoluble est recueilli par filtration, lavé à l'eau puis séché. Le résidu est ensuite recristallisé dans le méthanol à chaud. Après refroidissement, le précipité obtenu est recueilli par filtration, lavé avec un minimum de méthanol froid et séché, pour conduire au produit attendu.
*Point de fusion**: 112-114°C*

### EXEMPLE 3 : 7-Fluoro-4-(2-fluoroéthyl)-3,4-dihydro-2H-1,2,4-benzothiadiazine 1,1-dioxyde

### Stade A : 2-Amino-5-fluorobenzénesulfonamide

Le produit attendu est obtenu à partir de 4-fluoroaniline, selon le procédé décrit dans J. Chem. Soc. Perkin. I 1979, 1043-1047.

### Stade B : 7-Fluoro-4-(2-fluoroéthyl)-4H-1,2,4-benzothiadiazine 1, 1 -dioxyde

Le produit attendu est obtenu selon le procédé décrit dans les stades A et B de l'Exemple 1, à partir du composé obtenu au stade précédent.
*Point de fusion* *: 197-198°C*

### Stade C: 7-Fluoro-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion* : *133-134°C*

### EXEMPLE 4 : 6,7-Dichloro-4-(2-fluoroéthyl)-3,4-dihydro-2H-1,2,4-benzothiadiazine 1,1-dioxyde

### Stade A : 2-Amino-4,5-dichlorobenzènesulfonamide

Le produit attendu est obtenu à partir de 3,4-dichloroaniline, par chlorosulfonation selon le procédé décrit dans J. Med. Chem. 1971, 15, 118-120, suivie de la conversion du chlorure de sulfonyle obtenu en sulfonamide, à l'aide d'ammoniaque.

### Stade B : 6,7-Dichloro-4-(2-fluoroéthyl)-4H 1,2,4-benzothiadiazine 1,1-dioxyde

Le produit attendu est obtenu selon le procédé décrit dans les stades A et B de l'Exemple 1, à partir du composé obtenu au stade précédent.
*Point de fusion* *: 233-234°C*

### Stade C : 6,7-Dichloro-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 156-157°C*

### EXEMPLE 5 : 6-Chloro-7-fluoro-4-(2-fluoroéthyl)-3,4-dihydro-2H-1,2,4-benzothiadiazine 1,1-dioxyde

### Stade A : 6-Chloro-7-fluoro-4-(2-fluoroéthyl)-4H-1,2,4-benzothiadiazine 1,1-dioxyde

Le produit attendu est obtenu selon le procédé décrit dans les stades A et B de l'Exemple 4, à partir de 3-chloro-4-fluoroaniline.
*Point de fusion* *: 195-196°C*

### Stade B : 6-Chloro-7-fluoro-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 183-184°C*

### EXEMPLE 6 : 6-Chloro-4-(2-fluoroéthyl)-3,4-dihydro-2H-1,2,4-benzothiadiazine 1,1-dioxyde

### Stade A : 6-Chloro-4-(2-fluoroéthyl)-4H-1,2,4-benzothiadiazine 1,1-dioxyde

Le produit attendu est obtenu selon le procédé décrit dans les stades A et B de l'Exemple 3, à partir de 3-chloroaniline.
*Point de fusion**: 182-184°C*

### Stade B : 6-Chloro-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion* *: 171-175°C*

### EXEMPLE 7 : 6-Fluoro-4-(2-fluoroéthyl)-3,4-dihydro-2H-1,2,4-benzothiadiazine 1,1-dioxyde

### Stade A : 6-Fluoro-4-(2-fluoroéthyl)-4H-1,2,4-benzothiadiazine 1,1-dioxyde

Le produit attendu est obtenu selon le procédé décrit dans les stades A et B de l'Exemple 3, à partir de 3-fluoroaniline.
*Point de fusion* *: 155-160°C*

### Stade B : 6-Fluoro-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 107-109°C*

### EXEMPLE 8 : 7-Bromo-6-chloro-4-(2-fluoroéthyl)-3,4-dihydro-2H-1,2,4-benzothiadiazine 1,1-dioxyde

### Stade A : 7-Bromo-6-chloro-4-(2-fluoroéthyl)-4H 1,2,4-benzothiadiazine 1,1-dioxyde

Le produit attendu est obtenu selon le procédé décrit dans les stades A et B de l'Exemple 4, à partir de 4-bromo-3-chloroaniline.
*Point de fusion* *: 219-224°C*

### Stade B : 7-Bromo-6-chloro-4-(2-fluoroéthyl)-3,4-dihydro-2H 1,2,4-benzothiadiazine 1,1-dioxyde

Le produit attendu est obtenu selon le procédé décrit dans le stade C de l'Exemple 1, à partir du composé obtenu au stade précédent.
*Point de fusion* *: 159-162°C*

### EXEMPLE 9 : 4-(2-Fluoroéthyl)-7-méthyl-3,4-dihydro-2H-1,2,4-benzothiadiazine 1,1-dioxyde

### Stade A : 4-(2-Fluoroéthyl)-7-méthyl-4H 1,2,4-benzothiadiazine 1, 1 -dioxyde

Le produit attendu est obtenu selon le procédé décrit dans les stades A et B de l'Exemple 1, à partir de 2-amino-5-méthylbenzènesulfonamide, dont la préparation est décrite dans J. Chem. Soc. Perkin 11979,1043-1047.
*Point de fusion**: 179-184°C*

### Stade B : 4-(2-Fluoroéthyl)-7-méthyl-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion* : *88-90°C*

### EXEMPLE 10 : 7-Chloro-4-(4-fluorobutyl)-3,4-dihydro-2H-1,2,4-benzothiadiazine 1,1-dioxyde

### Stade A : 7-Chloro-4-(4-fluorobutyl)-4H-1,2,4-benzothiadiazine 1,1-dioxyde

150 mg du produit obtenu au stade A de l'Exemple 1 sont dissous dans 8 ml d'acétonitrile, additionné de 300 mg de K₂CO₃ et de 0,15 ml de 1-bromo-4-fluorobutane.
Le milieu réactionnel est placé en autoclave scellé et chauffé à 100°C durant 3h. Après refroidissement, le solvant est évaporé sous pression réduite et le résidu est repris par un minimum d'eau. L'insoluble est recueilli par filtration, lavé à l'eau et séché. Le précipité est ensuite recristallisé à chaud dans un minimum d'acétate d'éthyle. Après refroidissement, le produit titre est recueilli par filtration, lavé avec un minimum d'acétate d'éthyle froid et séché.
*Point de fusion**: 135-138°C*

### Stade B : 7-Chloro-4-(4-fluorobutyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 154-156°C*

### EXEMPLE 11 : 6,8-Dichloro-4-(2-fluoroéthyl)-3,4-dihydro-2H-1,2,4-benzothiadiazine 1,1-dioxyde

### Stade A : 6,8-Dichloro-4-(2-fluoroéthyl)-4H-1,2,4-benzothiadiazine 1,1-dioxyde

Le produit attendu est obtenu selon le procédé décrit dans les stades A et B de l'Exemple 3, à partir de 3,5-dichloroaniline.

### Stade B : 6,8-Dichloro-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion* *: 180-184°C*

### EXEMPLE 12 : 7-Chloro-4-(3-fluoropropyl)-3,4-dihydro-2H-1,2,4-benzothiadiazine 1,1- dioxyde

### Stade A : 7-Chloro-4-(3-fluoropropyl)-4H-1,2,4-benzothiadiazine 1,1-dioxyde

Le produit attendu est obtenu selon le procédé décrit dans le stade A de l'Exemple 10, en remplaçant le 1-bromo-4-fluorobutane par le 1-bromo-3-fluoropropane.
*Point de fusion* *: 209-212°C*

### Stade B : 7-Chloro-4-(3-fluoropropyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 143-145°C*

### EXEMPLE 13 : 7-Chloro-4-(fluorométhyl)-3,4-dibydro-2H-1,2,4-benzothiadiazine-1,1- dioxyde

### Stade A : 7-Chloro-4-(fluorométhyl)-4H-1,2,4-benzothiadiazine-1,1-dioxyde

Le produit attendu est obtenu selon le procédé décrit dans le stade A de l'Exemple 10, en remplaçant le 1-bromo-4-fluorobutane par le bromofluorométhane.
*Point de fusion* *: 227-232°C*

### Stade B : 7-Chloro-4-(fluorométhyl)-3,4-dihydro-2H-1,2,4-benzothiadiazine-1,1-dioxyde

Le produit attendu est obtenu selon le procédé décrit dans le stade C de l'Exemple 1, à partir du composé obtenu au stade précédent.

### EXEMPLE 14 : 7-Bromo-4-(2-fluoroéthyl)-3,4-dihydro-2H-1,2,4-benzothiadiazine 1,1- dioxyde

### Stade A : 7-Bromo-4-(2-fluoroéthyl)-4H-1,2,4-benzothiadiazine 1,1-dioxyde

Le produit attendu est obtenu selon le procédé décrit dans les stades A et B de l'Exemple 3, à partir de 4-bromoaniline.
*Point de fusion**: 229-232°C*

### Stade B : 7-Bromo-4-(2-fluoroéthyl)-3,4-dihydro-2H 1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 122-124°C*

### EXEMPLE 15 : 7-Iodo-4-(2-fluoroéthyl)-3,4-dihydro-2H-1,2,4-benzothiadiazine 1,1-dioxyde

### Stade A : 7-Iodo-4-(2-fluoroéthyl)-4H-1,2,4-benzothiadiazine 1,1-dioxyde

Le produit attendu est obtenu selon le procédé décrit dans les stades A et B de l'Exemple 3, à partir de 4-iodoaniline.
*Point de fusion**: 220-223°C*

### Stade B : 7-Iodo-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion* *: 156-158°C*

### EXEMPLE 16 : 4-(2-Fluoroéthyl)-7-nitro-3,4-dihydro-2H-1,2,4-benzothiadiazine 1,1-dioxyde

### Stade A : 4-(2-Fluoroéthyl)-7-nitro-4H 1,2,4-benzothiadiazine 1,1-dioxyde

Le produit attendu est obtenu selon le procédé décrit dans les stades A et B de l'Exemple 3, à partir de 4-nitroaniline.

### Stade B : 4-(2-Fluoroéthyl)-7-nitro-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 165-166°C*

### EXEMPLE 17 : 7-Nitro-4-(2,2,2-trifluoroéthyl)-3,4-dihydro-2H-1,2,4-benzothiadiazine 1,1-dioxyde

### Stade A: 7-Nitro-4-(2,2,2-trifluoroéthyl)-4H-1 ,2,4-benzothiadiazine 1,1-dioxyde

Le produit attendu est obtenu selon le procédé décrit dans les stades A et B de l'Exemple 3, à partir de 4-nitroaniline et de 1,1,1-trifluoro-2-iodoéthane.

### Stade B : 7-Nitro-4-(2,2,2-trifluoroéthyl)-3,4-dihydro-2H 1,2,4-benzothiadiazine 1,1-dioxyde

Le produit attendu est obtenu selon le procédé décrit dans le stade C de l'Exemple 1, à partir du composé obtenu au stade précédent.
*Point de fusion* *: 191-193°C*

### EXEMPLE 18 : 7-Chloro-4-(2,2-difluoroéthyl)-3,4-dihydro-2H-1,2,4-benzothiadiazine 1,1-dioxyde

### Stade A : 5-Chloro-2-(2,2-difluoroacétamido)benzènesulfonamide

A 1 g de 2-amino-5-chlorobenzènesulfonamide (obtenu selon J. Chem. Soc. Perkin I 1979, 1043-1047) dissous dans 4 ml de dioxane, on ajoute à froid (+ 5°C) 0,6 ml de pyridine et 0,6 ml de chlorure d'acide difluoroacétique. Le ballon est immédiatement fermé hermétiquement et placé sous forte agitation à température ambiante pendant 10 minutes. On élimine ensuite les solvants sous pression réduite. Le résidu solide est repris par de l'eau (12 ml), et l'insoluble est recueilli par filtration, lavé à l'eau et séché pour conduire au produit attendu.
*Point de fusion**: 180-181°C*

### Stade B : 5-Chloro-2-(2,2-difluoroéthylamino)benzènesulfonamide

1 g du composé obtenu au stade précédent sont mis en suspension dans 15 ml d'éther sec ; on y ajoute 500 mg de LiAlH₄. Le milieu est placé sous agitation. Après 30 minutes, le milieu est refroidi sur bain de glace, et additionné lentement d'eau ; le mélange est ensuite acidifié à pH 4 par ajout d'HCl concentré. Le milieu est extrait par de l'acétate d'éthyle; les phases organiques sont séchées puis évaporées sous pression réduite. Le résidu sec est ensuite recristallisé dans un mélange acétone-eau (1 : 10).
*Point de fusion**: 127-131°C*

### Stade C : 7-Chloro-4-(2,2-difluoroéthyl)-4H-1,2,4-benzothiadiazine-1,1-dioxyde

300 mg du composé obtenu au stade précédent sont mis en suspension dans 1,5 ml d'orthoformiate d'éthyle. Le mélange est chauffé en vase ouvert à 180°C pendant 45 minutes. Après refroidissement, l'insoluble qui apparaît est recueilli par filtration et lavé plusieurs fois au diéthyléther et séché.
*Point de fusion* *: 183-186°C*

### Stade D : 7-Chloro-4-(2,2-difluoroéthyl)-3,4-dihydro-2H-1,2,4-benzothiadiazine-1,1-dioxyde

250 mg du composé obtenu au stade précédent sont dissous à chaud dans 15 ml d'isopropanol. La solution est additionnée de 370 mg de NaBH₄ finement broyé et chauffée à 60°C durant 20 min. le solvant est ensuite évaporé sous pression réduite et le résidu est repris par 10 ml d'eau. La suspension obtenue est amenée à pH 5 à l'aide de HCl 6N et extraite 3 fois au chloroforme. La phase organique est séchée au sulfate de magnésium et évaporée sous pression réduite. Le résidu est ensuite recristallisé dans le méthanol à chaud. Après refroidissement, le précipité obtenu est recueilli par filtration, lavé au méthanol froid et séché.
*Point de fusion**: 126-128°C*

### EXEMPLE 19: 6,7-Difluoro-4-(2-fluoroéthyl)-3,4-dihydro-2H-1,2,4-benzothiadiazine 1,1-dioxyde

### Stade A : 6,7-Difluoro-4-(2-fluoroéthyl)-4H-1,2,4-benzothiadiazine 1,1-dioxyde

Le produit attendu est obtenu selon le procédé décrit dans les stades A et B de l'Exemple 3, à partir de 3,4-difluoroaniline.
*Point de fusion* *: 198-202°C*

### Stade B : 6,7-Difluoro-4-(2-fluoroéthyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion* *: 128-130°C*

### EXEMPLE 20 : 7-Chloro-2-éthylaminocarbonyl-4-(2-fluoroéthyl)-3,4-dihydro-2H-1,2,4-benzothiadiazine-1,1-dioxyde

A 500 mg du composé de l'Exemple 1 dissous dans 2,5 ml d'acétonitrile sont ajoutés 0,5 ml de triéthylamine et 2,5 ml d'isocyanate d'éthyle. Le milieu réactionnel est laissé sous agitation à température ambiante pendant 5 heures. Le solvant est ensuite éliminé sous pression réduite. Le résidu est repris par 10 ml d'acétone ; la solution obtenue est additionnée de charbon puis filtrée ; le filtrat est ensuite additionné d'eau. Le précipité obtenu est recueilli par filtration, lavé à l'eau et séché.
*Point de fusion* *: 138-140°C*

### EXEMPLE 21 : 6,7-Dichloro-4-(3-fluoropropyl)-3,4-dihydro-2H-1,2,4-benzothiadiazine 1,1-dioxyde

### Stade A : 6,7-Dichloro-4H-1,2,4-benzothiadiazine 1,1-dioxyde

Le produit attendu est obtenu selon le procédé décrit dans le stade A de l'Exemple 1 à partir du composé obtenu au stade A de l'Exemple 4.

### Stade B : 6,7-Dichloro-4-(3-fluoropropyl)-4H-1,2,4-benzothiadiazine 1,1-dioxyde

Le produit attendu est obtenu selon le procédé décrit dans le stade A de l'Exemple 12 à partir du composé obtenu au stade précédent.

### Stade C: 6,7-Dichloro-4-(3 fluoropropyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 180-182°C*

### EXEMPLE 22 : 6,7-Dichloro-4-(4-fluorobutyl)-3,4-dihydro-2H-1,2,4-benzothiadiazine 1,1-dioxyde

### Stade A : 6,7-Dichloro-4-(4-fluorobutyl)-4H-1,2,4-benzothiadiazine 1,1-dioxyde

Le produit attendu est obtenu selon le procédé décrit dans le stade A de l'Exemple 10, à partir du composé obtenu au stade A de l'Exemple 21.

### Stade B : 6,7-Dichloro-4-(4 fluorobutyl)-3,4-dihydro-2H-1,2,4-benzothiadiazine 1,1-dioxyde

Le produit attendu est obtenu selon le procédé décrit dans le stade C de l'Exemple 1, à partir du composé obtenu au stade précédent.
*Point de fusion**: 141-143°C*

### EXEMPLE 23: 4-(2-Fluoroéthyl)-7-(méthylsulfonyl)-3,4-dibydro-2H-1,2,4-benzothiadiazine 1,1-dioxyde

### Stade A : 4-(2-Fluoroéthyl)-7-(méthylsulfonyl)-4H-1,2,4-benzothiadiazine 1,1-dioxyde

Le produit attendu est obtenu selon le procédé décrit dans les stades A et B de l'Exemple 1, à partir de 2-amino-5-(méthylsulfonyl)-benzènesulfonamide.

### Stade B : 4-(2-Fluoroéthyl)-7-(méthylsulfonyl)-3,4-dihydro-2H-1,2,4-benzothiadiazine 1, 1-dioxyde

Le produit attendu est obtenu selon le procédé décrit dans le stade C de l'Exemple 1, à partir du composé obtenu au stade précédent.
*Point de fusion**: 193-195°C*

### EXEMPLE 24 : 6,7-Dichloro-4-(2,2-difluoroéthyl)-3,4-dihydro-2H-1,2,4-benzothiadiazine 1,1-dioxyde

Le produit attendu est obtenu selon le procédé de l'Exemple 18, en remplaçant au Stade A le 2-amino-5-chlorobenzénesulfonamide par le composé obtenu au stade A de l'Exemple 4.
*Point de fusion* *: 141-145°C*

### EXEMPLE 25 : 7-Chloro-4-(2,2-difluoroéthyl)-6-fluoro-3,4-dihydro-2H-1,2,4-benzothiadiazine 1,1-dioxyde

Le produit attendu est obtenu selon le procédé de l'Exemple 18, en remplaçant au Stade A le 2-amino-5-chlorobenzènesulfonamide par le 2-amino-5-chloro-4-fluorobenzènesulfonamide.
*Point de fusion**: 155-159°C*

### EXEMPLE 26: 8-Chloro-4-(2-fluoroéthyl)-3,4-dihydro-2H-1,2,4-benzothiadiazine 1,1-dioxyde

Le produit attendu est obtenu selon le procédé décrit dans l'Exemple 1, à partir de 2-amino-6-chlorobenzènesulfonamide et de 1-fluoro-2-iodoéthane.

### EXEMPLE 27: 4-(2-Fluoroéthyl)-7-(trifluorométhyl)-3,4-dibydro-2H-1,2,4-benzothiadiazine 1,1-dioxyde

Le produit attendu est obtenu selon le procédé décrit dans l'Exemple 1, à partir de 2-amino-5-(trifluorométhyl)-benzènesulfonamide et de 1-fluoro-2-iodoéthane.

### EXEMPLE 28: 4-(2-Fluoroéthyl)-7-méthoxy-3,4-dihydro-2H-1,2,4-benzothiadiazine 1,1-dioxyde

Le produit attendu est obtenu selon le procédé décrit dans l'Exemple 1, à partir de 2-amino-5-méthoxybenzènesulfonamide et de 1-fluoro-2-iodoéthane.

### EXEMPLE 29: 7-Chloro-4-(2,2,2-trifluoroéthyl)-3,4-dihydro-2H-1,2,4-benzothiadiazine 1,1-dioxyde

Le produit attendu est obtenu selon le procédé décrit dans l'Exemple 1, à partir de 2-amino-5-chlorobenzènesulfonamide et de 1,1,1-trifluoro-2-iodoéthane.

### EXEMPLE 30: 4-(2-Fluoroéthyl)-3,4-dihydro-2H-1,2,4-benzothiadiazine 1,1-dioxyde

Le produit attendu est obtenu selon le procédé décrit dans l'Exemple 1, à partir de 2-amino-benzènesulfonamide et de 1-fluoro-2-iodoéthane.

### EXEMPLE 31: 7,8-Dichloro-4-(2-fluoroéthyl)-3,4-dihydro-2H-1,2,4-benzothiadiazine 1,1-dioxyde

Le produit attendu est obtenu selon le procédé décrit dans l'Exemple 1, à partir de 2-amino-5,6-dichlorobenzènesulfonamide et de 1-fluoro-2-iodoéthane.
*Point de fusion* *: 230-232°C*

### EXEMPLE 32: 6,8-Difluoro-4-(2-fluoroéthyl)-3,4-dihydro-2H-1,2,4-benzothiadiazine 1,1-dioxyde

Le produit attendu est obtenu selon le procédé décrit dans l'Exemple 1, à partir de 2-amino-4,6-difluorobenzènesulfonamide et de 1-fluoro-2-iodoéthane.
*Point de fusion* *: 146-147°C*

### EXEMPLE 33: 5,8-Difluoro-4-(2-fluoroéthyl)-3,4-dihydro-2H-1,2,4-benzothiadiazine 1,1-dioxyde

Le produit attendu est obtenu selon le procédé décrit dans l'Exemple 1, à partir de 2-amino-3,6-difluorobenzènesulfonamide et de 1-fluoro-2-iodoéthane.
*Point de fusion**: 118-120°C*

### ETUDE PHARMACOLOGIOUE DES DERIVES DE L'INVENTION

### EXEMPLE 34 : Etude de l'effet des produits sur le courant ionique induit par l'AMPA dans les ovocytes de Xenopus

Des ARNm sont préparés à partir de cortex cérébral de rat mâle Wistar par la méthode de chlorure de césium/thiocyanate de guanidium. Les ARNm Poly (A+) sont isolés avec le kit PolyATtract mRNA Isolation systems (Promega, USA) et injectés à raison de 50 ng par ovocyte. Les ovocytes sont laissés 3 à 4 jours en incubation à 18°C pour permettre l'expression des récepteurs puis conservés à 4°C jusqu'à utilisation.

Les enregistrements électrophysiologiques sont réalisés à température ambiante dans une chambre en plexiglass perfusée en continu avec du milieu OR2 (J. Exp. Zool., 1973, 184, 321-334) par la méthode de « voltage-clamp » à 2 électrodes, une 3^{ème} électrode placée dans le bain sert de référence.

Tous les composés et l'AMPA sont appliqués *via* la perfusion. Le (S)-AMPA est utilisé à la concentration de 10 µM. Le courant ionique induit par l'application d'AMPA en absence et en présence de composés est mesuré. Pour chaque composé étudié, on définit la concentration doublant (EC2X) ou quintuplant (EC5X) l'intensité du courant induit par l'AMPA seul (5 à 50 nA).

Les composés de l'invention potentialisent fortement les effets excitateurs de l'AMPA. A titre d'exemple, le composé de l'Exemple 1 présente un EC2X de 6,7 ± 1,4 µM et un EC5X de 14,5 ± 0,6 µM.

### EXEMPLE 35 : Reconnaissance d'objet chez le rat Wistar adulte de 3 mois

Le test de reconnaissance d'objet chez le rat Wistar a été initialement développé par ENNACEUR et DECACOUR (Behav. Brain Res., 1988, 31, 47-59). Ce test est basé sur l'activité exploratoire spontanée de l'animal et possède les caractéristiques de la mémoire épisodique chez l'homme. Sensible au vieillissement (SCALL et coll., Eur. J. Pharmacol., 1997, 325, 173-180), ainsi qu'aux dysfonctionnements cholinergiques (BARTOLINI et coll., Pharm. Biochem. Behav. 1996, 53(2) , 277-283), ce test de mémoire est fondé sur l'exploration différentielle de 2 objets de forme assez similaire, l'un familier, l'autre nouveau. Préalablement au test, les animaux (rats Wistar mâles de 3 mois : CERJ, France) sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule par voie orale 60 minutes avant chaque session, explorent de façon identique l'objet familier et l'objet nouveau, ce qui signe l'oubli de l'objet déjà présenté. Les animaux traités par un composé facilitateur mnémocognitif, explorent de façon préférentielle l'objet nouveau, ce qui signe le souvenir de l'objet déjà présenté.
Les résultats obtenus montrent que les composés de l'invention augmentent la mémorisation de façon importante et à faible dose.

A titre d'exemple, le composé de l'exemple 1 améliore significativement la rétention mnésique pour des doses allant de 0,03 à 1 mg/kg par voie orale.

### EXEMPLE 36 : Reconnaissance d'objet chez le rat Wistar âgé de 17 mois

Chez le rat âgé, il existe un déficit de rétention mnésique dans le test de reconnaissance d'objet. Préalablement au test, les animaux ( rats Wistar mâles de 17 mois : CERJ, France) sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{ème} session (3 minutes), 1 heure plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{ème} session. Les animaux témoins, traités préalablement au véhicule par voie orale 60 minutes avant chaque session, explorent de façon identique l'objet familier et l'objet nouveau, ce qui signe l'oubli de l'objet déjà présenté. Les animaux traités par un composé facilitateur mnémocognitif, explorent de façon préférentielle l'objet nouveau, ce qui signe le souvenir de l'objet déjà présenté.
Les résultats obtenus montrent que les composés de l'invention s'opposent au déficit de rétention mnésique lié à l'âge dans le test de reconnaissance d'objet à faibles doses.
A titre d'exemple, le composé de l'exemple 1 s'oppose significativement au déficit de rétention mnésique lié à l'âge dans le test de rétention mnésique dès la dose de 0,1 mg/kg par voie orale.

### EXEMPLE 37 : Composition pharmaceutique

| Formule de préparation pour 1000 comprimés dosés à 10 mg : | |
|---|---|
| Composé de l'exemple 1 | 10g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 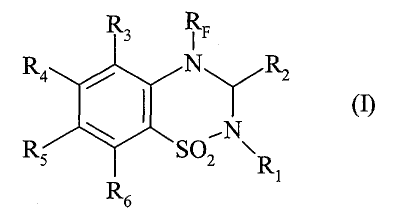 dans laquelle :
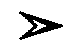 R_{F} représente un groupement mono- ou poly- fluoroalkyle C₁-C₆ linéaire ou ramifié, ou un groupement monofluoro- ou polyfluoro- cycloalkylalkyle, dans lequel la partie alkyle est en C₁-C₆ et est linéaire ou ramifiée, et la partie cycloalkyle est en C₃-C₇,
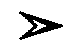 R₁ représente un atome d'hydrogène ou un groupement choisi parmi alkylaminocarbonyle C₁-C₆ linéaire ou ramifié et alkyle C₁-C₆ linéaire ou ramifié éventuellement substitué par un ou plusieurs atomes d'halogène,
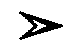 R₂ représente un atome d'hydrogène ou d'halogène ou un groupement choisi parmi cycloalkyle C₃-C₇ et alkyle C₁-C₆ linéaire ou ramifié éventuellement substitué par un ou plusieurs atomes d'halogène,
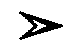 R₃, R₄, R₅ et R₆, identiques ou différents, représentent chacun un atome ou un groupement choisi parmi les atomes d'hydrogène, d'halogène et les groupements nitro, cyano, alkylsulfonyle C₁-C₆ linéaire ou ramifié, hydroxy, alkoxy C₁-C₆ linéaire ou ramifié, alkyle C₁-C₆ linéaire ou ramifié éventuellement substitué par un ou plusieurs atomes d'halogène, et amino éventuellement substitué par un ou deux groupements alkyle C₁-C₆ linéaire ou ramifié,
ainsi que leurs sels d'addition avec un acide ou une base pharmaceutiquement acceptable, et leurs isomères optiques lorsqu'ils existent,
étant entendu que R₃ représente un atome d'hydrogène lorsque R₆ ne représente pas un atome d'hydrogène.

2. Composé de formule (I) selon la revendication 1, **caractérisé en ce que** R_{F} représente un groupement monofluoroalkyle C₁-C₆ linéaire ou ramifié.

3. Composé de formule (I) selon la revendication 2, **caractérisé en ce que** R_{F} représente un groupement fluorométhyle, 2-fluoroéthyle, 3-fluoropropyle ou 4-fluorobutyle.

4. Composé de formule (I) selon la revendication 1, **caractérisé en ce que** R_{F} représente un groupement 2,2-difluoroéthyle ou 2,2,2-trifluoroéthyle.

5. Composé de formule (I) selon l'une quelconque des revendications 1 à 4, **caractérisé en ce que** R₁ représente un atome d'hydrogène.

6. Composé de formule (I) selon l'une quelconque des revendications 1 à 5, **caractérisé en ce que** R₂ représente un atome d'hydrogène ou de fluor.

7. Composé de formule (I) selon l'une quelconque des revendications 1 à 6, **caractérisé en ce que** R₃ représente un atome d'hydrogène.

8. Composé de formule (I) selon l'une quelconque des revendications 1 à 7, **caractérisé en ce que** R₄ représente un atome d'hydrogène ou d'halogène.

9. Composé de formule (I) selon l'une quelconque des revendications 1 à 8, **caractérisé en ce que** R₅ représente un atome d'halogène.

10. Composé de formule (I) selon l'une quelconque des revendications 1 à 9, **caractérisé en ce que** R₆ représente un atome d'hydrogène ou d'halogène.

11. Composé de formule (I) selon la revendication 1, qui est le 7-chloro-4-(2-fluoroéthyl)-3,4-dihydro-2*H*-1,2,4-benzothiadiazine 1,1-dioxyde.

12. Composé de formule (I) selon la revendication 1, qui est le 6,7-dichloro-4-(2-fluoroéthyl)-3,4-dihydro-2*H*-1,2,4-benzoth*iadiazine 1,1-dioxyde.

13. Composé de formule (I) selon la revendication 1, qui est le 6-chloro-7-fluoro-4-(2-fluoroéthyl)-3,4-dihydro-2*H*-1,2,4-benzothiadiazine 1,1-dioxyde.

14. Composé de formule (I) selon la revendication 1, qui est le 6-chloro-7-bromo-4-(2-fluoroéthyl)-3,4-dihydro-2*H*-1,2,4-benzothiadiazine 1,1-dioxyde.

15. Composé de formule (VI) : 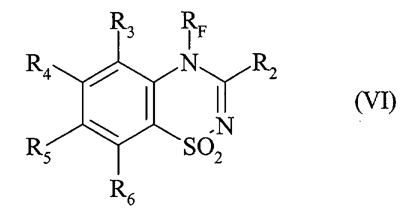 dans laquelle R₂, R₃, R₄, R₅, R₆ et R_{F} sont tels que définis dans la revendication 1.

16. Procédé de préparation des composés de formule (I) selon la revendication 1, à partir du composé de formule (II) : 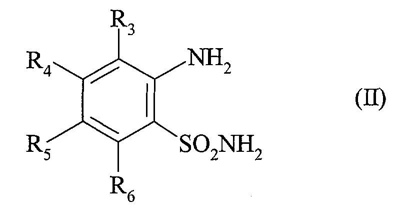 dans laquelle R₃, R₄, R₅ et R₆ sont tels que définis dans la formule (I),
que l'on cyclise en présence d'un composé de formule (III) :
R₂-C(OR₇)₃ (III)
dans laquelle R₂ est tel que défini dans la formule (I), et R₇ représente un groupement alkyle C₁-C₆ linéaire ou ramifié,
pour conduire au composé de formule (IV) : 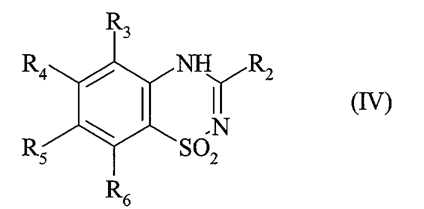 dans laquelle R₂, R₃, R₄, R₅ et R₆ sont tels que définis précédemment,
que l'on met en réaction avec un composé de formule (V) :
X ― R_{F} (V)
dans laquelle R_{F} est tel que défini dans la formule (I) et X représente un groupement partant choisi parmi les atomes d'iode, de brome et les groupements tosylate, mésylate et triflate,
pour conduire au composé de formule (VI) : 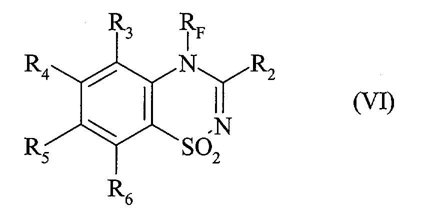 dans laquelle R₂, R₃, R₄, R₅, R₆ et R_{F} sont tels que définis précédemment,
que l'on met en réaction avec un agent réducteur, pour conduire au composé de formule (Ia), cas particulier des composés de formule (I) pour lequel R₁ représente un atome d'hydrogène : 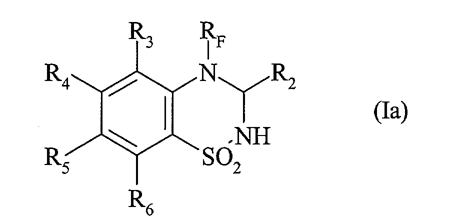 dans laquelle R₂, R₃, R₄, R₅, R₆ et R_{F} sont tels que définis précédemment,
composé de formule (Ia) que l'on met en réaction, lorsqu'on souhaite accéder aux composés de formule (I) pour lesquels R₁ représente un groupement alkyle éventuellement substitué,
avec un composé de formule (VII) :
X ― R'₁ (VII)
dans laquelle R'₁ représente un groupement alkyle C₁-C₆ linéaire ou ramifié éventuellement substitué par un ou plusieurs atomes d'halogène, et X représente un groupement partant choisi parmi les atomes d'iode, de brome et les groupements tosylate, mésylate et triflate,
pour conduire au composé de formule (Ib) : 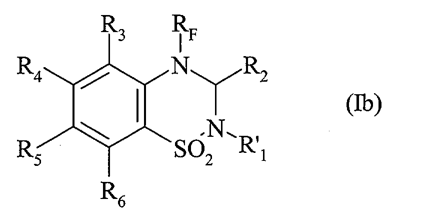 dans laquelle R₂, R₃, R₄, R₅, R₆, R_{F} et R'₁ sont tels que définis précédemment,
ou composé de formule (Ia) que l'on met en réaction, lorsqu'on souhaite accéder aux composés de formule (I) pour lesquels R₁ représente un groupement alkylaminocarbonyle,
avec un composé de formule (VIII) :
R₈-N=C=O (VIII)
dans laquelle R₈ représente un groupement alkyle C₁-C₆ linéaire ou ramifié,
pour conduire au composé de formule (Ic) : 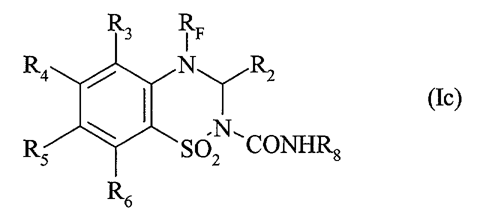 dans laquelle R₂, R₃, R₄, R₅, R₆, R_{F} et R₈ sont tels que définis précédemment,
les composés de formule (Ia), (Ib) et (Ic) constituant la totalité des composés de formule (I).

17. Composition pharmaceutique contenant comme principe actif un composé selon l'une quelconque des revendications 1 à 14, en combinaison avec un ou plusieurs véhicules inertes, non toxiques et pharmaceutiquement acceptables.

18. Utilisation des composés de formule (I) selon l'une quelconque des revendications 1 à 14 pour la fabrication de médicaments utiles en tant que modulateurs AMPA.

19. Utilisation des composés de formule (I) selon l'une quelconque des revendications 1 à 14 pour la fabrication de médicaments utiles pour le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.
